# EUROPEAN PATENT APPLICATION

(11) **EP 4 393 509 A1**
(43) Date of publication of application: **03.07.2024**
(21) Application number: 22861452.5
(22) Date of filing: 26.08.2022
(51) Int. Cl.: A61K 45/00, A61K 39/395, A61P 35/00, A61P 39/06, A61P 43/00

(54) **REACTIVE OXYGEN SPECIES (ROS) PRODUCTION PROMOTER**

(30) Priority: 26.08.2021 JP 2021138260
(71) Applicant: Perseus Proteomics Inc., Tokyo 103-0015 (JP)
(72) Inventor: KATSUMI Keiko, Tokyo 103-0015 (JP); OYA Junpei, Tokyo 103-0015 (JP); ISHII Keisuke, Tokyo 103-0015 (JP); SAKAMOTO Aya, Tokyo 103-0015 (JP)
(74) Representative: Hoffmann Eitle
(86) International application number: PCT/JP2022/032161
(87) International publication number: WO 2023/027164

(57) **Abstract**

It is an object of the present invention to provide an ROS production-enhancing agent. According to the present invention, an ROS production-enhancing agent, comprising a substance that recognizes a transferrin receptor, is provided.

## Description

### Technical Field

The present invention relates to an ROS production-enhancing agent, a ferritin degradation-promoting agent, a ferroptosis-inducing agent, and an apoptosis-inducing agent, each of which comprises a substance that recognizes a transferrin receptor.

### Background Art

Transferrin receptor (TfR) has been identified to be a receptor that is present on a reticulocyte as a cell membrane structure for incorporating transferrin (Tf)-bound iron into a cell. Thereafter, it has been discovered that TfR is expressed in placental trophoblasts, in activated lymphocytes, and further, in various tumor cells. For example, the high expression of TfR in breast cancer, prostate cancer, lung cancer, pancreatic cancer, colon cancer, stomach cancer, bladder cancer, hepatic cancer, cervical cancer, brain tumor, chronic lymphocytic leukemia, non-Hodgkin's lymphoma, and adult T-cell leukemia has been reported. Moreover, since TfR is expressed on the surface of various types of cancer cells at a high level and is expressed in normal cells at a low level, this receptor is recognized as a molecular target for cancer therapy. For example, Patent Document 1 discloses an antibody capable of specifically recognizing the transferrin receptor and an anti-cancer agent using the aforementioned antibody. Patent Document 2 discloses an iron uptake inhibitor for inhibiting iron uptake in cells, which comprises an antibody that recognizes the amino acids at positions 629 to 633 of a human transferrin receptor.

### Prior Art Document

### Patent Document

Patent Document 1: International Publication WO2014/073641
Patent Document 2: International Publication WO2020/105621

### Summary of Invention

### Object to be Solved by the Invention

As described above, it has been known that a substance that recognizes a transferrin receptor is used as an anticancer agent or is used as an iron uptake inhibitor. However, the influence of the substance that recognizes a transferrin receptor upon ferritin expression and ROS production in cells is unknown, and whether or not the substance that recognizes a transferrin receptor induces cell death due to ferroptosis and apoptosis via an increase in ROS production is also unknown. It is an object of the present invention to provide an ROS production-enhancing agent, a ferritin degradation-promoting agent, a ferroptosis-inducing agent, and an apoptosis-inducing agent.

### Means for Solving the Object

As a result of intensive studies conducted directed towards achieving the above-described object, the present inventors have found that a transferrin receptor-binding substance promotes degradation of ferritin and enhances ROS production, so that cell growth is suppressed or cell death is induced, thereby completing the present invention.

Specifically, according to the present invention, the following inventions are provided.
(1) An ROS production-enhancing agent, comprising a substance that recognizes a transferrin receptor.
(2) A ferritin degradation-promoting agent, comprising a substance that recognizes a transferrin receptor.
(3) A ferroptosis-inducing agent, comprising a substance that recognizes a transferrin receptor.
(4) An apoptosis-inducing agent, comprising a substance that recognizes a transferrin receptor.
(5) The agent according to any one of (1) to (4), wherein the substance that recognizes a transferrin receptor is an antibody that recognizes a transferrin receptor.
(6) The agent according to (5), wherein the antibody that recognizes a transferrin receptor is an antibody that recognizes a human transferrin receptor.
(7) The agent according to (6), wherein the antibody that recognizes a human transferrin receptor is an antibody that recognizes the amino acids at positions 629 to 633 of a human transferrin receptor, or an antibody that inhibits the binding of another antibody to the amino acids at positions 629 to 633 of a human transferrin receptor.
(8) The agent according to any one of (5) to (7), wherein the antibody is an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and also having a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.
(9) The agent according to any one of (5) to (8), wherein the antibody is an antibody having a heavy chain variable region as set forth in SEQ ID NO: 7 and a light chain variable region as set forth in SEQ ID NO: 8.
(10) The agent according to any one of (5) to (7), wherein the antibody is any one of:
   (a) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 11, 12, and 13, respectively, and also having a light chain first complementarity determining region (VL CDR1), a light chain second complementarity determining region (VL CDR2), and a light chain third complementarity determining region (VL CDR3), which are as set forth in SEQ ID NOs: 14, 15, and 16, respectively;
   (b) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 17, 18, and 19, respectively, and also having a light chain first complementarity determining region (VL CDR1), a light chain second complementarity determining region (VL CDR2), and a light chain third complementarity determining region (VL CDR3), which are as set forth in SEQ ID NOs: 20, 21, and 22, respectively;
   (c) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 23, 24, and 25, respectively, and also having a light chain first complementarity determining region (VL CDR1), a light chain second complementarity determining region (VL CDR2), and a light chain third complementarity determining region (VL CDR3), which are as set forth in SEQ ID NOs: 26, 27, and 28, respectively; and
   (d) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 29, 30, and 31, respectively, and also having a light chain first complementarity determining region (VL CDR1), a light chain second complementarity determining region (VL CDR2), and a light chain third complementarity determining region (VL CDR3), which are as set forth in SEQ ID NOs: 32, 33, and 34, respectively.
(11) The agent according to (10), wherein the antibody is any one of:
   an antibody having a heavy chain variable region as set forth in SEQ ID NO: 35 and a light chain variable region as set forth in SEQ ID NO: 36;
   an antibody having a heavy chain variable region as set forth in SEQ ID NO: 37 and a light chain variable region as set forth in SEQ ID NO: 38;
   an antibody having a heavy chain variable region as set forth in SEQ ID NO: 39 and a light chain variable region as set forth in SEQ ID NO: 40; and
   an antibody having a heavy chain variable region as set forth in SEQ ID NO: 41 and a light chain variable region as set forth in SEQ ID NO: 42.
(12) The agent according to any one of (5) to (11), wherein the antibody is a human antibody or a humanized antibody.
(13) The agent according to any one of (5) to (12), wherein the antibody is an antibody fragment selected from the group consisting of Fab, Fab', F(ab')₂, a single-chain antibody (scFv), a multi-specific antibody, a disulfide-stabilized V region (dsFv) and a peptide comprising CDR.

(A1) A method for enhancing ROS production, which comprises administering a substance that recognizes a transferrin receptor to a subject.
(A2) A method for promoting degradation of ferritin, which comprises administering a substance that recognizes a transferrin receptor to a subject.
(A3) A method for inducing ferroptosis, which comprises administering a substance that recognizes a transferrin receptor to a subject.
(A4) A method for inducing apoptosis, which comprises administering a substance that recognizes a transferrin receptor to a subject.
(B1) A substance that recognizes a transferrin receptor, which is for use in the treatment of enhancing ROS production.
(B2) A substance that recognizes a transferrin receptor, which is for use in the treatment of promoting degradation of ferritin.
(B3) A substance that recognizes a transferrin receptor, which is for use in the treatment of inducing ferroptosis.
(B4) A substance that recognizes a transferrin receptor, which is for use in the treatment of inducing apoptosis.
(C1) Use of a substance that recognizes a transferrin receptor for the production of an ROS production-enhancing agent.
(C2) Use of a substance that recognizes a transferrin receptor for the production of a ferritin degradation-promoting agent.
(C3) Use of a substance that recognizes a transferrin receptor for the production of a ferroptosis-inducing agent.
(C4) Use of a substance that recognizes a transferrin receptor for the production of an apoptosis-inducing agent.

### Advantageous Effects of Invention

According to the present invention, there are provided a novel ROS production-enhancing agent, a novel ferritin degradation-promoting agent, a novel ferroptosis-inducing agent, and a novel apoptosis-inducing agent.

### Brief Description of Drawings

[Fig.1] Fig. 1 shows the sites of individual TfR mutant fragments, at which point mutations are performed.
[Fig.2] Fig. 2 shows the reactivity of TfR436 with soluble wild-type TfR (sTfR) and TfR mutant fragments.
[Fig.3] Fig. 3 shows the Tf-TfR binding inhibitory activity of TfR436.
[Fig.4] Fig. 4 shows the growth-suppressing effect of TfR436.
[Fig.5] Fig. 5 shows the ferritin-degrading effect of TfR436.
[Fig.6] Fig. 6 shows the ROS production-increasing effect of TfR436.
[Fig.7] Fig. 7 shows the caspase 3/7 activity amount-increasing effect of TfR436.
[Fig.8] Fig. 8 shows the cell cycle arrest effect of TfR436.
[Fig.9] Fig. 9 shows the results obtained by examining a change in the expression level of ferritin by a plurality of TfR antibodies.
[Fig.10] Fig. 10 shows the results obtained by examining a change in the amount of ROS by a plurality of TfR antibodies.

### Embodiment of Carrying out the Invention

Hereinafter, the present invention will be described in more detail.

### Definitions and General Techniques

Unless otherwise specified in the present description, scientific terms used regarding the present invention include meanings that are generally understood by a person skilled in the art. In general, nomenclatures and techniques applied to the cell and tissue culture, molecular biology, immunology, microbiology, genetics, protein and nucleic acid chemistry, and hybridization, which are described in the present description, are well known in the present technical field, and thus, are commonly used.

The methods and techniques of the present invention are carried out in accordance with conventional methods that are well known in the present technical field, in such ways as described in a variety of general reference documents cited and discussed throughout the present description and more specific reference documents, unless otherwise specified.

### Ferritin degradation, enhancement of ROS production, and ferroptosis induction

Ferroptosis is a cell death mechanism, and it is considered that intracellular free iron (Fe²⁺) catalyzes a chain of peroxidation reactions of cell membrane phospholipids and lipid hydroxyl radicals are thereby accumulated, resulting in cell death. As mechanisms of occurrence of ferroptosis in cells, the following mechanisms are conceived: induction of autophagy, degradation of ferritin, an increase in Fe²⁺, an increase in ROS production, and induction of ferroptosis (cell death) (Jing Du et al, Free Radical Biology and Medicine 131 (2019) 356-369). In Example 4 as described later, it was confirmed that an antibody that recognizes a transferrin receptor decreases ferritin in cells. In addition, in Example 5, it was confirmed that such an antibody that recognizes a transferrin receptor increases ROS production in cells. Moreover, in Examples 3 and 8, it was confirmed that such an antibody that recognizes a transferrin receptor suppresses cell growth and decreases cell viability. The aforementioned results of Examples 3 to 5 and 8 suggest that ferritin degradation and an increase in ROS production occur as a mechanism of suppressing cell growth by an antibody that recognizes a transferrin receptor, and cell growth is thereby suppressed.

### Enhancement of mitochondrial ROS production, and apoptosis induction

The enhancement of ROS produced in mitochondria activates apoptosis signaling. There are two pathways for apoptosis, which are called endogenous pathway and exogenous pathway. In the endogenous pathway, apoptosis is activated by cell stress, DNA damage, developmental signals, and the removal of survival factors, whereas in the exogenous pathway, cell death receptors on the cell surface are activated by ligands such as Fas, TNFR1 and TRAIL, and finally, caspase-3 and caspase-7 are activated, so that apoptosis progresses, thereby inducing cell death. The results of Example 6 suggest that apoptosis is induced in certain cell lines.

### Arrest of cell cycle

Cells are divided into two daughter cells, through regular cycles called M-phase, G1-phase, S-phase, and G2-phase. A series of processes is called cell cycle. Since various iron-demanding enzymes function in the process, the cycle is arrested under circumstances in which iron supply is terminated, eventually leading to cell death. It is considered that the results of Example 7 indicate such a process.

### TfR

In the case of a human, transferrin receptor (TfR) is a single-pass transmembrane protein (SEQ ID NO: 9) composed of 760 amino acids, and it is encoded by human chromosome 3. This protein has also been known as a CD71 antigen, and it is considered that this protein is associated with incorporation of iron into cells and cell growth. The structure of the TfR of the present invention is not particularly limited, and thus, the TfR of the present invention includes all of a monomer, a polymer, an intact form expressed on a cell membrane, a soluble form constituted in an extracellular region, a truncated form, a mutation form caused by genetic mutation, deletion, etc., and a form that has undergone posttranslational modification by phosphorylation, and the like.

SEQ ID NO: 9
Met Met Asp Gln Ala Arg Ser Ala Phe Ser Asn Leu Phe Gly Gly Glu
Pro Leu Ser Tyr Thr Arg Phe Ser Leu Ala Arg Gln Val Asp Gly Asp
Asn Ser His Val Glu Met Lys Leu Ala Val Asp Glu Glu Glu Asn Ala
Asp Asn Asn Thr Lys Ala Asn Val Thr Lys Pro Lys Arg Cys Ser Gly
Ser Ile Cys Tyr Gly Thr Ile Ala Val Ile Val Phe Phe Leu Ile Gly
Phe Met Ile Gly Tyr Leu Gly Tyr Cys Lys Gly Val Glu Pro Lys Thr
Glu Cys Glu Arg Leu Ala Gly Thr Glu Ser Pro Val Arg Glu Glu Pro
Gly Glu Asp Phe Pro Ala Ala Arg Arg Leu Tyr Trp Asp Asp Leu Lys
Arg Lys Leu Ser Glu Lys Leu Asp Ser Thr Asp Phe Thr Gly Thr Ile
Lys Leu Leu Asn Glu Asn Ser Tyr Val Pro Arg Glu Ala Gly Ser Gln
Lys Asp Glu Asn Leu Ala Leu Tyr Val Glu Asn Gln Phe Arg Glu Phe
Lys Leu Ser Lys Val Trp Arg Asp Gln His Phe Val Lys Ile Gln Val
Lys Asp Ser Ala Gln Asn Ser Val Ile Ile Val Asp Lys Asn Gly Arg
Leu Val Tyr Leu Val Glu Asn Pro Gly Gly Tyr Val Ala Tyr Ser Lys
Ala Ala Thr Val Thr Gly Lys Leu Val His Ala Asn Phe Gly Thr Lys
Lys Asp Phe Glu Asp Leu Tyr Thr Pro Val Asn Gly Ser Ile Val Ile
Val Arg Ala Gly Lys Ile Thr Phe Ala Glu Lys Val Ala Asn Ala Glu
Ser Leu Asn Ala Ile Gly Val Leu Ile Tyr Met Asp Gln Thr Lys Phe
Pro Ile Val Asn Ala Glu Leu Ser Phe Phe Gly His Ala His Leu Gly
Thr Gly Asp Pro Tyr Thr Pro Gly Phe Pro Ser Phe Asn His Thr Gln

Phe Pro Pro Ser Arg Ser Ser Gly Leu Pro Asn Ile Pro Val Gln Thr
Ile Ser Arg Ala Ala Ala Glu Lys Leu Phe Gly Asn Met Glu Gly Asp
Cys Pro Ser Asp Trp Lys Thr Asp Ser Thr Cys Arg Met Val Thr Ser
Glu Ser Lys Asn Val Lys Leu Thr Val Ser Asn Val Leu Lys Glu Ile
Lys Ile Leu Asn Ile Phe Gly Val Ile Lys Gly Phe Val Glu Pro Asp
His Tyr Val Val Val Gly Ala Gln Arg Asp Ala Trp Gly Pro Gly Ala
Ala Lys Ser Gly Val Gly Thr Ala Leu Leu Leu Lys Leu Ala Gln Met
Phe Ser Asp Met Val Leu Lys Asp Gly Phe Gln Pro Ser Arg Ser Ile
Ile Phe Ala Ser Trp Ser Ala Gly Asp Phe Gly Ser Val Gly Ala Thr
Glu Trp Leu Glu Gly Tyr Leu Ser Ser Leu His Leu Lys Ala Phe Thr
Tyr Ile Asn Leu Asp Lys Ala Val Leu Gly Thr Ser Asn Phe Lys Val
Ser Ala Ser Pro Leu Leu Tyr Thr Leu Ile Glu Lys Thr Met Gln Asn
Val Lys His Pro Val Thr Gly Gln Phe Leu Tyr Gln Asp Ser Asn Trp
Ala Ser Lys Val Glu Lys Leu Thr Leu Asp Asn Ala Ala Phe Pro Phe
Leu Ala Tyr Ser Gly Ile Pro Ala Val Ser Phe Cys Phe Cys Glu Asp
Thr Asp Tyr Pro Tyr Leu Gly Thr Thr Met Asp Thr Tyr Lys Glu Leu
Ile Glu Arg Ile Pro Glu Leu Asn Lys Val Ala Arg Ala Ala Ala Glu
Val Ala Gly Gln Phe Val Ile Lys Leu Thr His Asp Val Glu Leu Asn
Leu Asp Tyr Glu Arg Tyr Asn Ser Gln Leu Leu Ser Phe Val Arg Asp
Leu Asn Gln Tyr Arg Ala Asp Ile Lys Glu Met Gly Leu Ser Leu Gln
Trp Leu Tyr Ser Ala Arg Gly Asp Phe Phe Arg Ala Thr Ser Arg Leu
Thr Thr Asp Phe Gly Asn Ala Glu Lys Thr Asp Arg Phe Val Met Lys
Lys Leu Asn Asp Arg Val Met Arg Val Glu Tyr His Phe Leu Ser Pro
Tyr Val Ser Pro Lys Glu Ser Pro Phe Arg His Val Phe Trp Gly Ser
Gly Ser His Thr Leu Pro Ala Leu Leu Glu Asn Leu Lys Leu Arg Lys
Gln Asn Asn Gly Ala Phe Asn Glu Thr Leu Phe Arg Asn Gln Leu Ala
Leu Ala Thr Trp Thr Ile Gln Gly Ala Ala Asn Ala Leu Ser Gly Asp
Val Trp Asp Ile Asp Asn Glu Phe

### React and Reactivity

The terms "react" and "reactivity" have the same meanings in the present description, unless otherwise specified. That is, these terms mean that an antibody recognizes an antigen. The antigen used herein may be any of an intact TfR expressed on a cell membrane, a truncated form, and a soluble form. In addition, the antigen may be either a TfR having a three-dimensional structure or a modified TfR. Examples of a means for examining reactivity include flow cytometry (FACS), enzyme-linked immunosorbent assay (ELISA), Western blotting, micro fluorescence measuring technique (FMAT), surface plasmon resonance (Biacore), immunostaining, and immunoprecipitation.

The antibody used in flow cytometry may be either an antibody labeled with a fluorescent substance such as FITC or with biotin, or an unlabeled antibody. A fluorescently-labeled avidin, a fluorescently-labeled anti-human immunoglobulin antibody, or the like is used, depending on the presence or absence of labeling of the antibody used and the type thereof. Reactivity can be evaluated by adding a sufficient amount of anti-TfR antibody (generally having a final concentration of 0.01 to 10 µg/mL) to an analyte, and then by comparing the obtained reactivity with the reactivity with a negative control antibody or a positive control antibody.

### Substance that recognizes a transferrin receptor

In the present invention, a substance that recognizes a transferrin receptor is used. Such a substance that recognizes a transferrin receptor is not particularly limited. For example, at least one selected from the group consisting of nucleic acids (nucleic acid aptamers, decoy nucleic acids, etc.), ribozymes, antibodies and fragments thereof, peptides, cyclic peptides, and peptide mimics, can be used. For example, aptamers that mimic transferrin are described in Wilner et al., Molecular Therapy-Nucleic Acids (2012) 1, e21; doi:10.1038/mtna.2012.14. On the other hand, peptides that target transferrin receptors are described in Jae H. Lee et al., Eur. J. Biochem. 268, 2004-2012 (2001). In the present invention, such nucleic acids, peptides and the like can be used.

### Antibody

In the present description, the following abbreviations (in the parentheses) are used in accordance with the customs, as necessary.
Heavy chain (H chain), light chain (L chain), heavy chain variable region (VH), light chain variable region (VL), complementarity determining region (CDR), first complementarity determining region (CDR1), second complementarity determining region (CDR2), third complementarity determining region (CDR3), heavy chain first complementarity determining region (VH CDR1), heavy chain second complementarity determining region (VH CDR2), heavy chain third complementarity determining region (VH CDR3), light chain first complementarity determining region (VL CDR1), light chain second complementarity determining region (VL CDR2), and light chain third complementarity determining region (VL CDR3).

In the present description, the term "antibody" has the same definitions as immunoglobulin, and should be understood as generally known in the present technical field. Specifically, the term "antibody" is not limited by any given specific method for producing the antibody. For example, the term "antibody" includes, but is not limited to, a recombinant antibody, a monoclonal antibody, and a polyclonal antibody.

In the present description, the term "human antibody" is used to mean any given antibody, in which the sequences of a variable region and a constant region are human sequences. This term includes antibodies which have human sequences and are modified, for example, to remove cysteine that may cause a possible decrease in immunogenicity, an increase in affinity, and undesirable folding. This term also includes antibodies produced in non-human cells by recombination, which enable glycosylation that is not specific to human cells. These antibodies can be prepared in various ways.

In the present description, the term "humanized antibody" means a non-human-derived antibody, in which amino acid residues characteristic for a non-human antibody sequence are substituted with residues found in positions corresponding to those of a human antibody. This "humanization" process is considered to reduce the immunogenicity of the obtained antibody in human. It would be understood that a non-human-derived antibody can be humanized using a technique well known in the present technical field. Please refer to, for example, Winter et al., Immunol. Today 14: 43-46 (1993). The target antibody can be produced by an engineering approach via a recombination DNA technique of substituting CH1, CH2, CH3, a hinge domain, and/or a framework domain with those of the corresponding human sequence. For example, WO92/02190, and U. S. Patent Nos. 5,530,101, 5,585,089, 5,693,761, 5,693,792, 5,714,350 and 5,777,085 can be referred. In the present description, the term "humanized antibody" includes a chimeric human antibody and a CDR-grafted antibody, within the definitions thereof.

The sequence of a framework region (FR) in a variable region of the antibody is not particularly limited, unless it substantially affects the specific binding ability of the antibody to the corresponding antigen. The FR region of a human antibody is preferably used, but it is also possible to use FR regions of animal species other than humans (e.g. a mouse or a rat).

In one aspect, the antibody comprises a constant region as well as a variable region (e.g. IgG antibody). The sequence of such a constant region is not particularly limited. For example, the constant region of a known human antibody can be used. The heavy chain constant region (CH) of a human antibody is not particularly limited, as long as it belongs to a human immunoglobulin (hereinafter referred to as "hIg"). Those of hIgG class are preferable, and any one of subclasses belonging to hIgG class, such as hIgG1, hIgG2, hIgG3 or hIgG4, may be used. On the other hand, the light chain constant region (CL) of a human antibody is not particularly limited, as long as it belongs to hIg, and those of κ class or λ class can be used. In addition, constant regions of animal species other than humans (e.g. a mouse or a rat) can also be used.

In the present description, the term "modified body" or "modified antibody" is used to mean that the amino acid sequence of the variable region (CDR sequences and/or FR sequences) of a parent antibody comprises a substitution, deletion, addition and/or insertion of one or multiple amino acids.

In the present description, the "parent antibody" means a TfR436 antibody that has a VH comprising the amino acid sequence as set forth in SEQ ID NO: 7 and a VL comprising the amino acid sequence as set forth in SEQ ID NO: 8. In the amino acid sequence, one or several (for example, 1 to 8, preferably 1 to 5, more preferably 1 to 3, and particularly preferably 1 or 2) amino acids are deleted, added, substituted and/or inserted. As a method of preparing the amino acid sequence of an antibody having a binding ability to TfR, which has been well known to a person skilled in the art, a method of introducing a mutation into a protein has been known. For instance, such a skilled person could prepare a modified antibody functionally equivalent to an antibody having a TfR-binding activity by appropriately introducing a mutation into the amino acid sequence of the antibody having a TfR-binding activity according to a site-directed mutagenesis (Hashimoto-Gotoh, T, Mizuno, T, Ogasahara, Y, an DNA Kagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci USA. 82, 488-492). Thus, an antibody comprising a mutation of one or several amino acids in the variable region or constant region of a parent antibody and having a binding activity to TfR can also be used.

In the present description, the phrase "an activity equivalent to the activity of the parent antibody" is used to mean that the binding activity of a certain antibody to human TfR is equivalent to that of the parent antibody thereof. The term "equivalent" does not necessarily mean the same level of activity. The activity may be increased, or the activity may also be decreased, as long as the antibody has the activity. An antibody having a decreased activity may be an antibody having an activity that is, for example, 30% or more, preferably 50% or more, more preferably 80% or more, further preferably 90% or more, and particularly preferably 95% or more of the activity of the original antibody.

The term "binding activity" means the activity of an antibody to recognize an antigen. This antigen may be an intact TfR expressed on a cell membrane, a truncated form, or a soluble form. In addition, the antigen may be either a TfR having a three-dimensional structure or a modified TfR. Examples of a means for examining the binding activity include flow cytometry (FACS), enzyme-linked immunosorbent assay (ELISA), Western blotting, micro fluorescence measuring technique (FMAT), and surface plasmon resonance (Biacore).

The Tf-TfR binding inhibitory activity of an antibody can be measured according to the method described later in "Example 2(2) Comparison between TfR436 antibody and antibodies of other companies in terms of Tf-TfR binding inhibition." That is, a TfR solution is dispensed into a substrate (a 96-well plate, etc.) and is then left at rest to obtain a solid phase, and thereafter, blocking is carried out. Subsequently, an HRP-labeled Tf solution is dispensed into the substrate, and an antibody is further added thereto, so that they are allowed to react with each other at room temperature. Thereafter, the substrate is washed, and a coloring reagent (TMB, etc.) is then added thereto, so that they are allowed to react with each other. After that, the absorbance is measured using a plate reader. By performing the above-described operations, the Tf-TfR binding inhibitory activity of the antibody can be evaluated.

The antibody is not limited by its origin, and it may be an antibody derived from any animal, such as a human antibody, a mouse antibody, or a rat antibody. Also, the present antibody may be a chimeric antibody or a humanized antibody. One preferred aspect of the antibody of the present invention is a human antibody.

The antibodies may be different from one another in terms of amino acid sequence, molecular weight, isoelectric point, the presence or absence of a sugar chain or the form thereof, etc., depending on the after-mentioned cells or hosts that produce the antibodies, or a purification method. For example, an antibody that undergoes a posttranslational modification on the amino acid sequence described in the present description is included in the present invention. Moreover, an antibody that undergoes a posttranslational modification on a site other than those for the known posttranslational modification is also included in the present invention. Furthermore, when the antibody is allowed to express in prokaryotic cells such as *Escherichia coli,* a methionine residue is added to the N-terminus of the amino acid sequence of the original antibody. Such an antibody may also be used in the present invention. An antibody that undergoes a posttranslational modification on a site other than those for the known posttranslational modification is also included in the present invention.

### Production of Antibody

Either a monoclonal antibody or a polyclonal antibody may be used as an antibody in the present invention. Such a monoclonal antibody and a polyclonal antibody can be produced by methods known to those skilled in the art.

Examples of such antibodies may include antibodies produced in animal blood, antibodies produced by hybridomas, antibodies produced by a host that is transformed with an expression vector containing an antibody gene according to a genetic engineering method, antibodies obtained by screening optimal antibodies from a clone library according to phage display and then producing the genes of the antibodies in CHO cells, and antibodies directly obtained as human antibodies, using transgenic mice that produce such human antibodies.

In order to produce a polyclonal antibody, serum is obtained by immunizing an animal such as a rabbit with an antigen. The obtained serum is purified, for example, by ammonium sulfate precipitation, protein A column, protein G column, DEAE ion exchange chromatography, or affinity column, so as to prepare a polyclonal antibody.

In order to produce a monoclonal antibody, an animal is immunized with an antigen, together with an adjuvant, as desired. After confirming that a desired antibody level has increased in the serum of the immunized animal, immune cells (splenic cells, etc.) are harvested from the animal, and the harvested cells are then fused with mammalian myeloma cells. Hybridomas obtained by the cell fusion can be selected by culturing them in a normal selective culture medium, such as, for example, a HAT culture medium (i.e. a culture medium containing hypoxanthine, aminopterin and thymidine). Thereafter, a limiting dilution method is carried out, so that hybridomas producing antibodies of interest can be screened.

Production of an antibody according to a phage display method will be described below.

### (1) Obtaining of scFv reacting with antigen using phage display library

Using a phage display technique, a library comprising a repertoire of antibodies having various affinity for TfR can be provided. Subsequently, such a library can be screened to identify and isolate antibodies against TfR. Preferably, the phage library is a scFv phage display library that is generated using human VL and VH cDNA that has been prepared from mRNA isolated from human B cells. A method of preparing and screening such a library is known in the present technical field. A genetic substance is recovered from phage clones exhibiting reactivity that have been screened using TfR as an antigen. By analyzing the selected phage gene, the DNA sequences of VH and VL encoding the variable region of a human antibody binding to the antigen can be determined. Using this scFv sequence, IgG is prepared from scFv, so as to obtain a human antibody.

### (2) Preparation of IgG from scFv (preparation of human antibody)

An H chain or L chain expression vector is produced, and it is then allowed to express in a host cell. Thereafter, the secreted supernatant is recovered and is then purified, so as to obtain a human antibody. Alternatively, such a human antibody can also be obtained by allowing VH and VL to express in a single vector (tandem type). These methods are well known, and can be carried out with reference to WO92/01047, WO92/20791, WO93/06213, WO93/11236, WO93/19172, WO95/01438, WO95/15388, WO97/10354, etc.

Specifically, DNA encoding VH is ligated to another DNA molecule encoding a heavy chain constant region (CH1, CH2 and CH3), so as to obtain a full-length heavy chain gene. The sequence of a human heavy chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The heavy chain constant region may be the constant region of IgG1, IgG2, IgG3, IgG4, IgA, IgE, IgM or IgD. The most preferred constant region is that of IgG1 or IgG2. The constant region sequence of IgG1 may include any given various alleles or allotypes known to be generated among different individuals, such as Gm (1), Gm (2), Gm (3) or Gm (17). These allotypes correspond to a substitution of amino acids naturally-occurring in the constant region of IgG1.

DNA encoding VL is ligated to another DNA molecule encoding the light chain constant region CL, so as to obtain a full-length L chain gene (and a Fab light chain gene). The sequence of a human light chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The light chain constant region may be the constant region of κ or λ. The κ constant region may include any given various alleles known to be generated among different individuals, such as Inv (1), Inv (2) or Inv (3). The λ constant region may be derived from any one of the three λ genes.

The thus obtained H chain- or L chain-encoding DNA is inserted into a vector to produce an expression vector, and the produced expression vector is then allowed to express in a host cell. Thereafter, the secreted supernatant is recovered and purified to obtain a human antibody. Examples of the expression vector include a plasmid, retrovirus, adenovirus, adeno-associated virus (AAV), plant viruses such as cauliflower mosaic virus or tobacco mosaic virus, a cosmid, YAC, and EBV-derived episome. An expression vector and an expression regulatory sequence are selected, so that they are suitable for a host cell used for expression. An antibody light chain gene and an antibody heavy chain gene can be inserted into different vectors, or the two genes can also be inserted into a single expression vector. An antibody gene is inserted into an expression vector by a standard method (for example, ligation of a complementary restriction site on an antibody gene fragment to a vector, or blunt-ended ligation applied when no restriction sites are present).

A favorable vector encodes a functionally complete human CH or CL immunoglobulin sequence having a suitable restriction site, which has been produced by an engineering approach such that any given VH or VL sequence can be easily inserted and then expressed therein, as described above. In such a vector, splicing generally takes place between a splice donor site in the inserted J region and a splice acceptor site preceding a human C domain, or such splicing also takes place in a splice region existing in a human CH exon. Polyadenylation and transcription termination take place in a natural chromosomal site downstream of a coding region. A recombinant expression vector can also encode a signal peptide that promotes the secretion of an antibody chain derived from a host cell. An antibody chain gene can be cloned into a vector, such that a signal peptide can be ligated in-frame to the amino terminus of an immunoglobulin chain. The signal peptide may be either an immunoglobulin signal peptide or a heterogeneous signal peptide (namely, it may be a non-immunoglobulin protein-derived signal peptide).

An expression vector for the antibody may also have sequences such as a sequence for regulating replication of the vector in a host cell (e.g. a replication origin) or a selective marker gene sequence, as well as an antibody gene and a regulatory sequence. The selective marker gene promotes selection of a host cell into which a vector has been introduced. For instance, the selective marker generally imparts resistance to drugs such as G418, hygromycin or methotrexate to a host cell into which the vector has been introduced. Preferred selective marker genes include a dihydrofolate reductase (DHFR) gene (used in selection/amplification of methotrexate as a dhfr-host cell), a neomycin phosphotransferase gene (used in selection of G418), and a glutamate synthase gene.

A host cell is transformed with an antibody gene expression vector produced by the above-described method. Any type of cell may be used as a host cell, as long as it can produce the present antibody. Examples of such a host cell include bacteria, yeast, animal cells, insect cells, and plant cells. Among these cells, animal cells are preferable. Examples of the animal cells include Chinese hamster ovary cells CHO/dhfr(-) and CHO/DG44, monkey-derived cells COS (A. Wright & S. L. Morrison, J. Immunol. 160, 3393-3402 (1998)), and SP2/O cells (mouse myeloma) (K. Motmans et al., Eur. J. Cancer Prev. 5, 512-5199 (1996), R. P. Junghans et al., Cancer Res. 50, 1495-1502 (1990)). For transformation, a lipofectin method (R. W. Malone et al., Proc. Natl. Acad. Sci. USA 86, 6007 (1989), P. L. Felgner et al., Proc. Natl. Acad. Sci. USA 84, 7413 (1987)), an electroporation method, a calcium phosphate method (F. L. Graham & A. J. van der Eb, Virology 52, 456-467 (1973)), a DEAE-Dextran method, and the like are preferably applied.

A transformant is cultured, and a human antibody is then separated from the cells of the transformant or a culture medium thereof. For separation/purification of the antibody, methods such as centrifugation, ammonium sulfate fractionation, salting-out, ultrafiltration, affinity chromatography, ion exchange chromatography and gel filtration chromatography can be used by appropriately combining them.

### Antibody Fragments

An antibody fragment can be produced based on the present antibody, or based on the sequence information of a gene encoding the present antibody. Examples of the antibody fragment include Fab, Fab', F(ab')₂, scFv, and dsFv antibodies.

Fab is obtained by digesting IgG by papain in the presence of cysteine. It is an antibody fragment with a molecular weight of approximately 50,000, which is constituted with L chain and H chain variable regions, and an H chain fragment consisting of a CH1 domain and a portion of a hinge region. In the present invention, the above-described antibody can be obtained by papain digestion. In addition, Fab can also be prepared by incorporating DNA encoding a portion of the H chain and the L chain of the above-described antibody into a suitable vector, then performing transformation with the resulting vector, and then obtaining it from the transformant .

Fab' is an antibody fragment with a molecular weight of approximately 50,000, which is obtained by cleaving a disulfide bond between the H chains of the below-mentioned F(ab')₂. In the present invention, Fab' can be obtained by digesting the above-described antibody by pepsin, and then cleaving a disulfide bond with a reducing agent. In addition, as with Fab, Fab' can also be prepared by genetic engineering using DNA encoding the Fab'.

F(ab')₂ is an antibody fragment with a molecular weight of approximately 100,000, which is obtained by binding, via a disulfide bond, one fragment (Fab') constituted with L chain and H chain variable regions and an H chain fragment consisting of a CH1 domain and a portion of a hinge region, to the other fragment (Fab'). In the present invention, F(ab')₂ can be obtained by digesting the above-described antibody by pepsin. In addition, as with Fab, F(ab')₂ can also be prepared by genetic engineering using DNA encoding the F(ab')₂.

scFv is an antibody fragment obtained by ligating the C-terminus of one chain of Fv consisting of an H chain variable region and an L chain variable region to the N-terminus of the other chain thereof, using a suitable peptide linker, so as to form a single chain. (GGGGS)₃ having high flexibility can be used, for example, as such a peptide linker. For instance, DNA encoding the H chain variable region and L chain variable region of the above-described antibody and DNA encoding a peptide linker are used to construct DNA encoding a scFv antibody, and the thus constructed DNA is then incorporated into a suitable vector. Thereafter, scFv can be prepared from a transformant obtained by transformation with the aforementioned vector.

dsFv is an Fv fragment obtained by introducing a Cys residue into a suitable site in each of an H chain variable region and an L chain variable region, and then stabilizing the H chain variable region and the L chain variable region by a disulfide bond. The site in each chain, into which the Cys residue is to be introduced, can be determined based on a conformation predicted by molecular modeling. In the present invention, for example, a conformation is predicted from the amino acid sequences of the H chain variable region and L chain variable region of the above-described antibody, and DNA encoding each of the H chain variable region and the L chain variable region, into which a mutation has been introduced based on such prediction, is then constructed. The thus constructed DNA is incorporated into a suitable vector. Thereafter, dsFv can be then prepared from a transformant obtained by transformation with the aforementioned vector.

Besides, it is also possible to ligate the scFv antibody to the dcFv antibody or the like using a suitable linker, or to fuse such an antibody fragment with streptavidin, so as to multimerize the antibody fragment.

### Multi-specific Antibody

The antibody may be a multi-specific antibody that recognizes TfR and simultaneously recognizes other targets. In particular, a bispecific antibody is often used.

The bispecific antibody means an antibody in which a molecule that recognizes TfR is combined with a molecule targeting another molecule.

Examples of the bispecific antibody may include bispecific (mab)₂ obtained by chemically crosslinking two molecules of monoclonal antibodies to each other, bispecific F(ab')2 obtained by chemically crosslinking two molecules of Fab fragments to each other, quadroma, bsDb (a bispecific diabody), scBsDb (a single-chain bispecific diabody), scBsTaFv (a single-chain bispecific tandem variable domain), Bite (a bispecific T cell Engager antibody), and DNL-F(ab)3 (docl-and-lock trivalent Fab) (Shim, H. Bispecific Antibodies and Antibody-Drug Conjugates for Cancer Therapy: Technological Considerations. Biomolecules 2020, 10, 360), but the forms of the bispecific antibody are not limited thereto.

### Pharmaceutical Composition and Preparation

A pharmaceutical composition and a preparation, each comprising the ROS production-enhancing agent, ferritin degradation-promoting agent, ferroptosis-inducing agent or apoptosis-inducing agent of the present invention, are also included in the scope of the present invention.

The ROS production-enhancing agent, ferritin degradation-promoting agent, ferroptosis-inducing agent or apoptosis-inducing agent of the present invention can be used to treat a subject who is in need of ROS production enhancement, ferritin degradation promotion, ferroptosis induction, or apoptosis induction, with an ROS production-enhancing agent, a ferritin degradation-promoting agent, a ferroptosis-inducing agent or an apoptosis-inducing agent.

The pharmaceutical composition or the preparation, each comprising the ROS production-enhancing agent, ferritin degradation-promoting agent, ferroptosis-inducing agent or apoptosis-inducing agent of the present invention, preferably comprises a physiologically acceptable diluent or carrier, in addition to the substance that recognizes a transferrin receptor. Examples of a suitable carrier used herein may include a normal saline, a phosphate buffered saline, a phosphate buffered saline with glucose, and a buffered saline, but the examples of the suitable carrier are not limited thereto. Otherwise, the substance that recognizes a transferrin receptor is freeze-dried or is frozen, and when needed, the aforementioned buffered aqueous solution may be added thereto to reconstitute the substance that recognizes a transferrin receptor, and the thus reconstituted substance that recognizes a transferrin receptor may be then used. The dosage form may be oral administration using tablets, capsules, granules, powdery agents, syrups, etc., or parenteral administration using injections (subcutaneous, intravenous, intramuscular, and intraperitoneal injections, etc.), transdermal, transmucosal, nasal, and transpulmonary administrations, suppository, etc.

The applied dose of the ROS production-enhancing agent, ferritin degradation-promoting agent, ferroptosis-inducing agent or apoptosis-inducing agent of the present invention is different depending on symptoms, age, body weight, etc. In general, in the case of oral administration, the present agent is administered at a dose of approximately 0.001 mg to 1,000 mg per day per adult, in terms of the amount of a substance that recognizes a transferrin receptor. Such a dose can be administered once or divided over several administrations per day. On the other hand, in the case of parenteral administration, the present agent can be administered at a dose of approximately 0.001 mg to 1,000 mg for a single administration, via subcutaneous, intramuscular, or intravenous administration.

The present invention will be described in more detail in the following examples. However, these examples are not intended to limit the scope of the present invention.

### Examples

In the following examples, the TfR436 antibody described in paragraphs 0090 and 0091 of International Publication WO2014/073641 was used.

The CDR sequences of the TfR436 antibody are shown below.
VH CDR1: SYGMH (SEQ ID NO: 1)
VH CDR2: VISYDGSNKYYADSVKG (SEQ ID NO: 2)
VH CDR3: DSNFWSGYYSPVDV (SEQ ID NO: 3)
VL CDR1: TRSSGSIASNSVQ (SEQ ID NO: 4)
VL CDR2: YEDTQRPS (SEQ ID NO: 5)
VL CDR3: QSYDSAYHWV (SEQ ID NO: 6)

The VH sequence and VL sequence of the TfR436 antibody are shown below.
TfR436 VH (SEQ ID NO: 7) 
DVQLVQSGGGVVQPGRSLRLSCAASGFPFKSYGMHWVRQAPGKGLEWVAV
ISYDGSNKYYADSVKGRFTISRDNSKNTLYLQMNSLRGEDTAVYYCARDSNF
WSGYYSPVDVWGQGTTVTVSS
TfR436 VL (SEQ ID NO: 8)

NFMLTQPHSVSESPGKTVTISCTRSSGSIASNSVQWYQQRPGSAPITVIYEDTQ
RPSGVPDRFSGSIDSSSNSASLTISGLQTEDEADYYCQSYDSAYHWVFGGGTK
LAVL

Moreover, in Examples 9 and 10, the TfR007 antibody, TfR014 antibody, TfR017 antibody and TfR018 antibody described in paragraph 0083 of International Publication No. WO2012/153707 were used.

The CDR sequences of the TfR007 antibody, the TfR014 antibody, the TfR017 antibody and the TfR018 antibody are shown below.
TfR007
   VH CDR1: SEQ ID NO: 11, VH CDR2: SEQ ID NO: 12, and VH CDR3: SEQ ID NO: 13
   VL CDR1: SEQ ID NO: 14, VL CDR2: SEQ ID NO: 15, and VL CDR3: SEQ ID NO: 16
TfR014
   VH CDR1: SEQ ID NO: 17, VH CDR2: SEQ ID NO: 18, and VH CDR3: SEQ ID NO: 19
   VL CDR1: SEQ ID NO: 20, VL CDR2: SEQ ID NO: 21, and VL CDR3: SEQ ID NO: 22
TfR017
   VH CDR1: SEQ ID NO: 23, VH CDR2: SEQ ID NO: 24, and VH CDR3: SEQ ID NO: 25
   VL CDR1: SEQ ID NO: 26, VL CDR2: SEQ ID NO: 27, and VL CDR3: SEQ ID NO: 28
TfR018
   VH CDR1: SEQ ID NO: 29, VH CDR2: SEQ ID NO: 30, and VH CDR3: SEQ ID NO: 31
   VL CDR1: SEQ ID NO: 32, VL CDR2: SEQ ID NO: 33, and VL CDR3: SEQ ID NO: 34
SEQ ID NO: 11: SYWLS
SEQ ID NO: 12: KIDPSDSYTQYSPSFEG
SEQ ID NO: 13: HGYDAFHV
SEQ ID NO: 14: SGSSSNIGNNAVN
SEQ ID NO: 15: YDDLLPS
SEQ ID NO: 16: AAWDDSLNGWV
SEQ ID NO: 17: ELSMH
SEQ ID NO: 18: GFDPEDGETIYAQKFQG
SEQ ID NO: 19: DAYYGSGSPRDAFDI
SEQ ID NO: 20: GGDNVGGKSLH
SEQ ID NO: 21: DDRDRPS
SEQ ID NO: 22: QVWDDISRLVI
SEQ ID NO: 23: DYAMY
SEQ ID NO: 24: GINWNSAIIGYADSVKG
SEQ ID NO: 25: EALYYSAFFDS
SEQ ID NO: 26: SGSSSNIGNNYVS
SEQ ID NO: 27: DNNKRPS
SEQ ID NO: 28: GTWDSSLSAWV
SEQ ID NO: 29: DYAMH
SEQ ID NO: 30: GINWNGGSTDYADSVEG
SEQ ID NO: 31: DYADLGSGSDY
SEQ ID NO: 32: SGSRSNIGSNYVH
SEQ ID NO: 33: RNDQRPS
SEQ ID NO: 34: ASWDDKMSGRL

The VH sequence and VL sequence of the TfR007 antibody are shown below.
TfR007 VH (SEQ ID NO: 35) 
EVQLVESGAEVKKPGESLKISCKGSGYTFTSYWLSWVRQLPGKGLEWMGKI
DPSDSYTQYSPSFEGQVTISADKSSNTAYLQWSSLKASDTAIYYCARHGYDA
FHVWGQGTTVTVSR
TfR007 VL (SEQ ID NO: 36)

QSVLTQPPSVSEAPRQRVTISCSGSSSNIGNNAVNWYQQLPGKAPKLLISYDD
LLPSGVSDRFSGSTSGTSASLAISGLQSEDEADYYCAAWDDSLNGWVFGGG
TKLTVLGQPKAAPSVTLFPPSSEELQANKATLVCLISDFYPGAVTVAWKADG
SPVKAGVETTKPSKQSNNKYAASSYLSLTPEQWKSHRSYSCQVTHEGSTVE
KTVAPTEC

The VH sequence and VL sequence of the TfR014 antibody are shown below.
TfR014 VH (SEQ ID NO: 37) 
QVQLQQSGAEVKKPGASVKVSCKVSGYTLTELSMHWVRQAPGKGLEWMG
GFDPEDGETIYAQKFQGRVTMTEDTSTDTAYMELSSLRSEDTAVYYCATDA
YYGSGSPRDAFDIWGQGTTVTVSR
TfR014 VL (SEQ ID NO: 38)

SYVLTQPPSVSVAPGETATITCGGDNVGGKSLHWYQVKPGQAPVLVVFDDR
DRPSGIPDRFSGANSRDTAALTISRVEAGDEADYYCQVWDDISRLVIFGGGT
RLTVLRQPKAAPSVTLFPPSSEELQANKATLVCLISDFYPGAVTVAWKADSS
PVKAGVETTTPSRQSNNKYAASSYLSLTPEQWKSHRSYSCQVTHEGSTVEK
TVAPTEC

The VH sequence and VL sequence of the TfR017 antibody are shown below.
TfR017 VH (SEQ ID NO: 39) 
EVQLVESGGGLVQPGGSLRLSCAASGFTFDDYAMYWVRQAPGKGLEWVSG
INWNSAIIGYADSVKGRFTISRDNSKNTLYLQMNSLRVEDTAVYFCAKEALY
YSAFFDSWGQGTLVTVSR
TfR017 VL (SEQ ID NO: 40)

QSVLTQPPSVSAAPGQKVTISCSGSSSNIGNNYVSWYQQLPGTAPKLLIYDN
NKRPSGIPDRFSGSRSGTSATLGITGLQTGDEADYYCGTWDSSLSAWVVGG
GTKLTVLGQPKAAPSVTLFPPSSEELRANKATLVCLISDFYPGAVTVAWKAD
SSPVKAGVETTTPSKQSNNKYAASSYLSLTPEQWKSHRSYSCQVTHEGSTVE
KTVAPTEC

The VH sequence and VL sequence of the TfR018 antibody are shown below.
TfR018 VH (SEQ ID NO: 41) 
EVQLVESGGGLVQPGRSLRLSCAASGFTFGDYAMHWVRQSPGKGLEWVSG
INWNGGSTDYADSVEGRFTISRDNAANSLFLQMNSLRAEDTAIYYCARDYA
DLGSGSDYWGQGTLVTVSS
TfR018 VL (SEQ ID NO: 42)

QSVLTQPPSASGTPGQRVTISCSGSRSNIGSNYVHWYQQLPGAAPKLLIYRND
QRPSGVPDRFSGSRSDTSAFLAISGLRSEDEADYYCASWDDKMSGRLFGGGT
KVTVLSQPKAAPSVTLFPPSSEELRANKATLVCLISDFYPGAVTVAWKADSSP
VKAGVETTTPSKQSNNKYAASSYLSLTPEQWKSHRSYSCQVTHEGSTVEKT
VAPTEC

### Example 1: Identification of binding site of TfR436 antibody

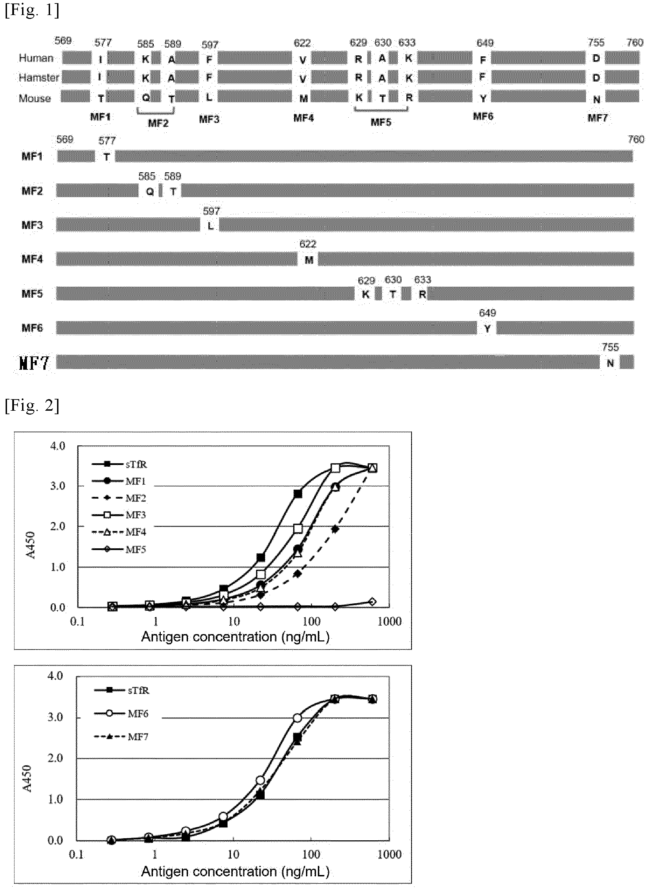

The TfR436 antibody did not cross-react with mouse TfR, but it exhibited cross-reactivity with hamster TfR. The amino acid sequence of a transferrin (TF)-binding site (the amino acids at positions 569 to 760) in TfR was aligned. The amino acids of a human TfR sequence, which were the same as hamster but were different from mouse, were picked up. The picked amino acids were subjected to point mutation, as shown in Fig. 1, so that soluble TfR mutant fragments were produced.

### (1) Production of soluble wild-type TfR (sTfR) and TfR mutant fragments (MF1 to MF7)

A human TfR extracellular domain (the amino acids at positions 89 to 760) or each TfR mutant fragment (MF1 to MF7) as shown in Fig. 1, and a nucleotide sequence encoding AAARGGPEQKLISEEDLNSAVDHHHHHH (SEQ ID NO: 10) were totally synthesized. A neomycin resistance gene and a DHFR gene were incorporated into the expression vector pCAGGS (Non-Patent Document 2.: Niwa et al. 1991), and thereafter, the synthesized genes were each inserted into the multicloning site of the obtained expression vector, so as to produce a pCAGGS-Neo-DHFR-sTFR-myc-his expression plasmid. Using Expifectamine (Thermo Fisher Scientific), the Expi293 cells (Thermo Fisher Scientific) were transfected with the above-described plasmid, and were then cultured at 37°C in 8% CO₂ at 135 rpm for 5 days. Thereafter, a culture supernatant was recovered by centrifugation. A HisTrapHP (Cytiva) column was connected with AKTA prime (Cytiva), and sTfR or MF1 to MF7 were then purified using 20 mM Imidazole/DPBS as a binding buffer and 500 mM Imidazole/DPBS as an elution buffer. The eluted protein was subjected to buffer exchange with 30 mM HEPES, 5% trehalose, pH 7.2, by using a Zeba spin column (Thermo Fisher Scientific).

### (2) Identification of binding site of TfR436 antibody

The thus purified sTfR or MF1 to MF7 were diluted with PBST (Phosphate Buffered Saline with Tween 20, TaKaRa), and the diluted solutions were prepared at 7 stages by 3-fold dilution from 600 ng/mL. Thereafter, the diluted solution was dispensed into Ni-NTA HisSorb Strips 96-well plate (QIAGEN) in an amount of 100 µL/well, and the plate was then placed on a shaker, followed by performing a reaction at room temperature. One hour later, the reaction mixture was washed with PBST Buffer 5 times, and the TfR436 antibody (1 ug/mL) was dispensed into the 96-well plate in an amount of 100 µL/well. The plate was placed on a shaker, and a reaction was then performed at room temperature for 1 hour. Thereafter, the reaction mixture was washed with PBS-T Buffer 5 times, and a 50,000-fold diluted secondary antibody, F(ab')2 Fragment Anti-Human IgG Fcy (Jackson Immuno Research), was then dispensed into the 96-well plate in an amount of 100 µL/well, followed by performing a reaction at room temperature for 1 hour. The reaction mixture was washed with PBST Buffer 5 times, and TMB Soluble Reagent (High Sensitivity) (Scy Tek) was then dispensed into the plate in an amount of 100 µL/well, followed by performing a reaction at room temperature in a dark place for 3 minutes. After that, TMB Stop Buffer (Scy Tek) was added to the plate in an amount of 100 µL/well, and the obtained mixture was then shaken using a shaker for 1 minute. Thereafter, the absorbance at 450 nm (ref. 620 nm) was measured using a plate reader.

As a result, as shown in Fig. 2, a reduction in the reactivity of the TfR436 antibody with the TfR mutant fragment MF5 was observed, but a reduction in the reactivity of the TfR436 antibody with other mutant fragments was not observed. That is to say, when the amino acids at positions 629, 630 and 633 of TfR are substituted with other amino acids, the TfR436 antibody cannot recognize the TfR. Thus, it was suggested that the amino acids at positions 629 to 633 of TfR should be an epitope recognized by the TfR436 antibody.

### Example 2: Comparison between TfR436 antibody and comparative antibodies in terms of Tf-TfR binding inhibition

### (1) Production of comparative antibody A24

Patent Document US2008/0193453 discloses an A24 antibody reacting against human TfR. In order to compare the TfR436 antibody with the A24 antibody, the deposited hybridomas were acquired, and the antibody was produced. Specifically, the hybridomas were seeded into RPMI1640 (GIBCO) supplemented with 10% FBS to a cell concentration of 1 to 2 × 10⁵/mL, and were then cultured in a 5% CO₂ incubator at 37°C. After completion of an expansion culture, the cells were recovered by centrifugation, and were then washed with PBS twice. Thereafter, the resulting cells were further subjected to an expansion culture in a serum-free medium Cosmedium 005 (Cosmo Bio Co., Ltd.) and 0.5% Nutridoma-CS (Roche) to result in a volume of 550 mL. Five days after the cells became confluent, a culture supernatant was recovered by centrifugation.

The recovered supernatant was applied onto a protein A carrier (Ab-Capcher ExTra: ProteNova), and an antibody binding to protein A was eluted with a 0.1 M glycine-HCl buffer (pH 2.7), and then, was rapidly neutralized with a 1 M Tris-HCl buffer (pH 8.5). Thereafter, using an Ultracell Ultrafiltration Disk (Merck), the buffer was exchanged with PBS.

### (2) Comparison between TfR436 antibody and antibody of other company in terms of Tf-TfR binding inhibition

The sTfR described in Example 1 was adjusted to a concentration of 5.0 µg/mL by addition of PBST, and the diluted solution was then dispensed into MaxiSorp 96-well plate (Nunc) in an amount of 100 µL/well. Thereafter, it was left at rest at 4°C overnight to obtain a solid phase. On the following day, the solid phase solution was discarded, and 100% Block Ace (DS Pharma Biomedical) was added to the plate in an amount of 200 µL/well, followed by leaving the obtained mixture at rest at room temperature so as to carry out blocking. One hour later, the reaction mixture was washed with PBST Buffer 5 times, and HRP-labeled Tf (2 ug/mL) was then dispensed into the plate in an amount of 50 µL/well. Thereafter, the TfR436 antibody, the A24 antibody (2-fold dilution series from 10 µg/mL), or holo-Tf (Merck) (2-fold dilution series from 300 µg/mL) was further added to the mixture in an amount of 50 µL/well. The obtained mixture was reacted at room temperature for 1 hour, and was then washed with PBST Buffer 5 times. Thereafter, TMB Soluble Reagent (High Sensitivity) was dispensed into the plate in an amount of 100 µL/well, and the obtained mixture was then reacted at room temperature in a dark place. Twenty five minutes later, TMB Stop Buffer was added to the plate in an amount of 100 µL/well, the obtained mixture was then shaken with a shaker for 1 minute, and the absorbance at 450 nm (ref. 620 nm) was then measured using a plate reader.

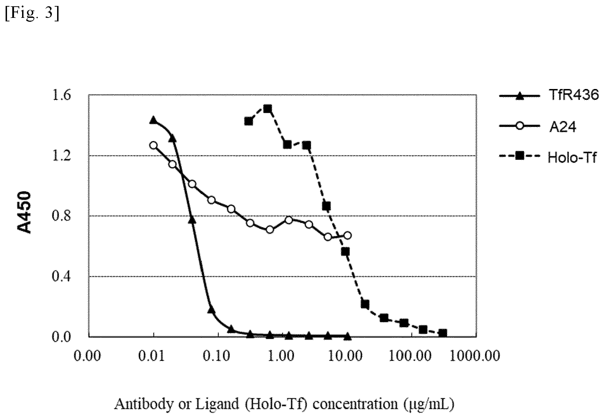

As a result, as shown in Fig. 3, the TfR436 antibody completely inhibited the binding of Tf-TfR at a significantly low dose (100 ng/mL). In contrast, the A24 antibody could not completely inhibit the binding of Tf-TfR, even though it was used at a dose of 10 µg/mL, and could inhibit only 50% of the Tf-TfR binding. Thus, it was suggested that the TfR436 antibody should be excellent in terms of inhibition of the Tf-TfR binding.

### Example 3: Growth-suppressing effect of TfR436 antibody

The growth-suppressing effect of a TfR436 antibody obtained by exposure to the TfR436 antibody was verified. Specifically, MV4;11, Kasumi-1, and HNT-34 were each suspended in each type of optimal medium for cell culture to result in a cell density of 1000 cells/50 µL, and were then seeded on a 96-well plate. The TfR436 antibody and a negative control monoclonal antibody were each diluted with the same medium as described above to 9 levels, so that a common ratio became 3.16 from a final concentration of 5 µg/mL (33.3 nM). These antibody-added media and antibody-not-added media were added in an amount of 50 µL each to individual cell lines. The thus obtained mixtures were cultured at 37°C in a 5% CO₂ incubator for 96 hours, and thereafter, 20 µL of CellTiter 96 AQueous One Solution Cell Growth Assay (Promega #G3580) was added to and blended with the culture in each well. The obtained mixtures were left at rest at 37°C in a 5% CO₂ incubator for 4 hours, and the absorbance at 505 nm was then detected. The well, to which only the medium had been added, was used as a blank. The mean value in the antibody-not-added well was set to be a growth percentage of 100%, and the growth percentage in each antibody concentration was calculated.

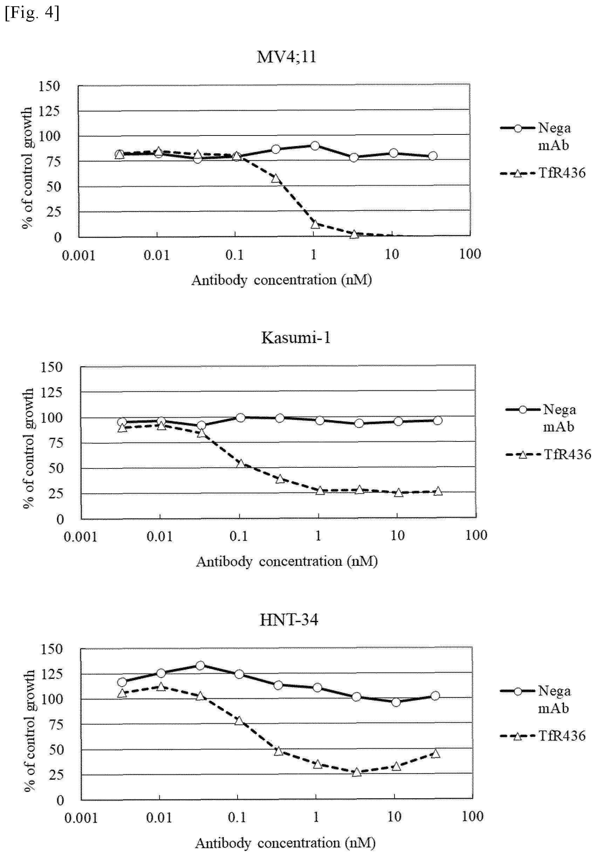

The results are shown in Fig. 4. It was confirmed that the TfR436 antibody suppresses the growth of each cell line, compared with the negative control monoclonal antibody (Nega Ab).

### Example 4: Change in ferritin amount by the TfR436 antibody

A change in the amount of ferritin caused by exposure to the TfR436 antibody was verified by a Western blotting method. Specifically, MV4;11, Kasumi-1, and HNT-34 were each suspended in each type of optimal medium for cell culture to result in a cell density of 1.0 × 10⁵ cells/mL, and were then seeded in a T75 flask or a T150 flask. The flask into which the TfR436 antibody or the negative control monoclonal antibody had been added, and the antibody-not-added flask, were cultured at 37°C in a 5% CO₂ incubator. It is to be noted that the final concentration of the TfR436 antibody was adjusted depending on the GI50 value of each cell line calculated from the growth percentage in each TfR436 antibody concentration of Example 3, and that the final concentration of the negative control monoclonal antibody was adjusted to the same concentration of the TfR436 antibody. The cells were recovered at 4, 24, 48, and 96 hours after the addition of the antibody, and were then washed with PBS. Thereafter, each cell line (1.0 × 10⁶ cells) was suspended in 100 µL of Complete Lysis-M EDTA-free (Roche #4719964001), and was then left at rest on ice for 30 minutes. After the cells had been centrifuged, a supernatant was recovered, and protein quantification was then carried out using Pierce BCA Protein Assay Kit (Thermo Fisher Scientific #23225). One-fifth volume of 5 × Sample Buffer (containing 2-mercaptoethanol) was added to and blended with the recovered supernatant, and the obtained mixture was then treated at 100°C for 3 minutes to prepare a sample. The prepared sample was applied in an amount of 7.5 µL (5 µg) each to a 5-20% gradient polyacrylamide gel, and was then electrophoresed. Thereafter, a protein was transcribed onto a PVDF membrane (Cytiva #RPN303F) by applying a voltage of 80 V for 60 minutes, using a transcription buffer (Tris-glycine buffer) containing 20% methanol. The transcribed PVDF membrane was blocked, using a blocking buffer in which 4 g of Block Ace powders (DS Pharma Biomedical #UK-B500) were dissolved in 100 mL of purified water. The following primary antibodies were diluted with a blocking buffer, and were then incubated at 4°C overnight.

### Primary antibodies (clone names) (source, product number, dilution factor)

### (1) Ferritin Heavy chain (B-12) (Santa Cruz Biotechnology, #sc-376594, 1000-fold)

### (2) Anti-Actin, clone C4 (Merck, #MAB1501, 5000-fold)

After the incubation, the membrane was washed with 0.05% -PBST at room temperature for 5 minutes three times. The secondary antibody ECL Anti-Mouse IgG HRP-Linked Whole Antibody (Cyriva, #NA931) was diluted with a blocking buffer to (1) 2500-fold and (2) 5000-fold, followed by performing incubation at room temperature for 60 minutes.

After the incubation, the membrane was washed with 0.05% -PBST at room temperature for 5 minutes three times. Using ECL Prime Western Blotting Detection Reagents (Cytiva #RPN2232), the sample was exposed and detected with Lumino-Image Analyzer LAS-3000 (FUJIFILM).

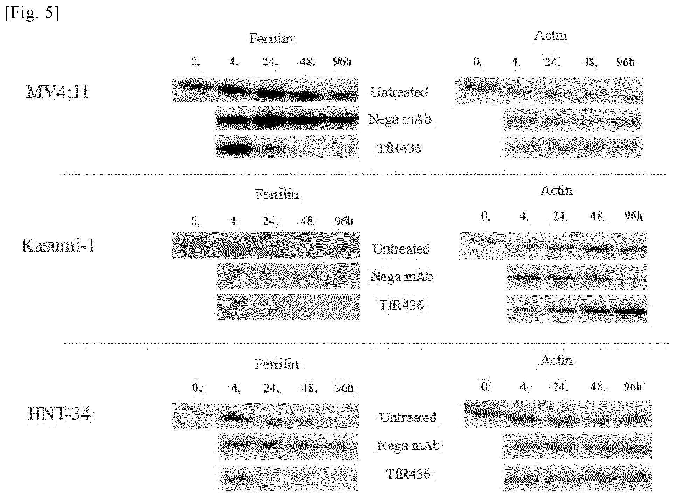

The results are shown in Fig. 5. It was confirmed that the TfR436 antibody decreases the amount of ferritin in each cell line, compared with non-addition of the antibody (Untreated) and the negative control monoclonal antibody (Nega mAb).

### Example 5: Measurement of ROS

ROS in mitochondria was measured using MitoSOX (Thermo Fisher Scientific). The cells were seeded at a cell density of 1 × 10⁵ cells/mL on a 6-well plate or a 60-mm dish. The TfR436 antibody was added to the cells, so that the final concentration of K562, MV4;11 or U266B1 became 5 µg/mL, and the final concentration of CCRF-CEM became 0.05 µg/mL, or the TfR436 antibody was not added to the cells. Thereafter, the resulting cells were cultured. After the cells had been cultured for a predetermined period of time, the cells were recovered and were then washed with HBSS twice. Thereafter, the cells were counted. The cells were seeded at a cell density of 1 × 10⁵ cells/well on a V-bottom 96-well plate, and were then stained with 5 µM MitoSOX at 37°C for 10 minutes. The resulting cells were washed with HBSS twice, and were then transferred onto a plate for fluorescence measurement. The fluorescence was measured at Ex 505 nm and Em 590 nm, using Infinite 200 Pro manufactured by Tecan. The fluorescence value of the solvent was subtracted from the measured value, and then, while the case of non-addition of the antibody was set to be 100%, the ROS production percentage in the case of addition of the TfR436 antibody was calculated.

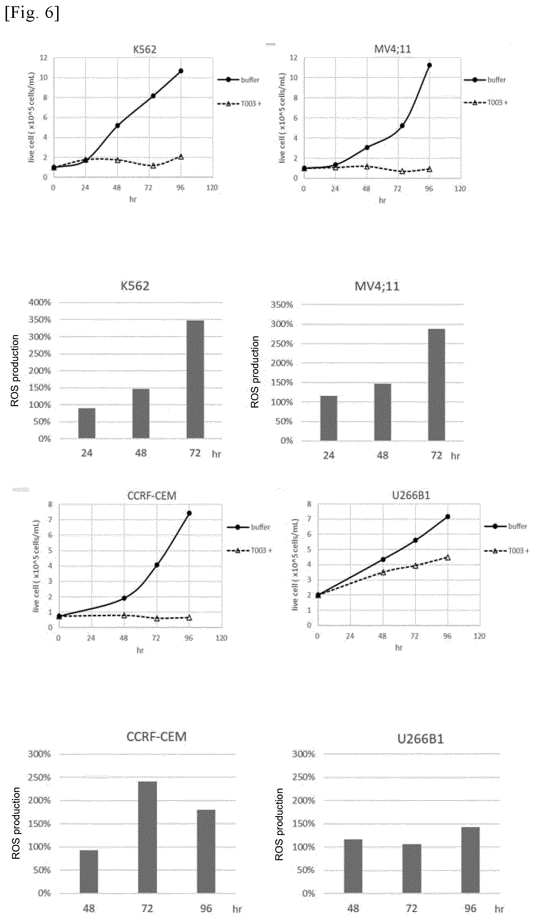

The results are shown in Fig. 6. It was confirmed that the TfR436 antibody increases ROS production in K562, MV4;11, and CCRF-CEM.

### Example 6: Measurement of amount of caspase 3/7 activity

The amount of caspase 3/7 activity caused by exposure to the TfR436 antibody was verified. K562, MV4;11, CCRF-CEM, and U266B1 cells were each suspended in each type of optimal medium for cell culture to result in a cell density of 10000 cells/50 µL, and were then seeded on a 96-well plate. The TfR436 antibody and the negative control monoclonal antibody were each diluted with the same medium as described above to 6 or 7 levels, respectively, so that a common ratio became 3.16 from a final concentration of 5 µg/mL (33.3 nM). These antibody-added media and antibody-not-added media were added in an amount of 50 µL each to individual cell lines. The thus obtained mixtures were cultured at 37°C in a 5% CO₂ incubator for 72 hours (K562 and MV4;11) or for 96 hours (CCRF-CEM and U266B1), and thereafter, 100 µL of Apo-ONE Homogeneous Caspase-3/7 Assay (Promega #G7790) was added to and blended with the culture in each well. The obtained mixtures were left at rest at room temperature for 1 to 2 hours, and thereafter, the amount of a fluorescent product, which indicates the amount of caspase 3/7 activity, was detected at an excitation wavelength of 465 nm and a fluorescence wavelength of 535 nm.

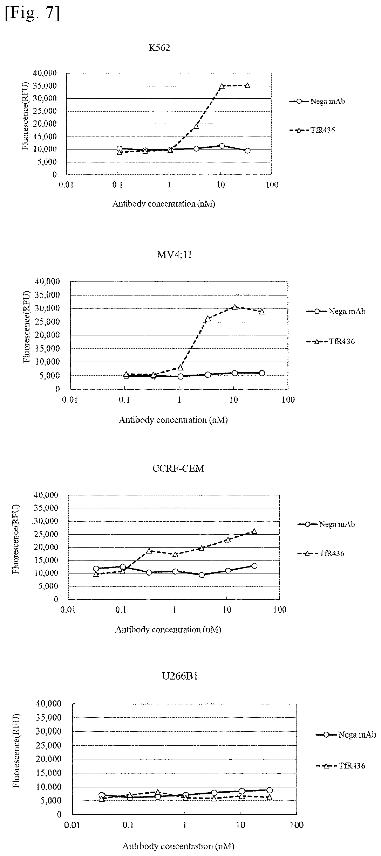

The results are shown in Fig. 7. It was confirmed that the TfR436 antibody increases the amount of caspase 3/7 activity in K562, MV4;11, and CCRF-CEM.

### Example 7: Arrest of cell cycle by TfR436 antibody

The influence of the TfR436 antibody on cell cycle was verified. HEL cells were prepared to have a cell density of 2 × 10⁶ cells/mL, and were then seeded on a 6-well plate in an amount of 1 mL/well. A TfR436 antibody-added medium was prepared to have a final concentration of 0.5, 1.0 or 2.0 µg/mL, whereas a negative control antibody-added medium was prepared to have a final concentration of 20 µg/mL. These antibody-added media or antibody-not-added media were added in an amount of 1 mL/well to individual wells. The thus obtained mixtures were cultured at 37°C in a 5% CO₂ incubator for 48 hours, and the cells were then peeled with trypsin and were recovered. The recovered cells were washed with PBS, and 1 mL of 70% EtOH was then added to the cells, so that the cells were immobilized. The cells were preserved at -20°C for 24 hours, and EtOH was then discarded. The cells were washed with PBS and were then counted. The cell cycle was measured by adding 0.5 mL of Fx Cycle PI/RNase Staining Solution (Thermo Fisher Scientific #F10797) to the cells, then leaving the resulting cells in a dark place for 30 minutes, and then measuring the cells using BD FACS Calibur Flow Cytometer (BD Biosciences). Thereafter, 50,000 cells were analyzed with FlowJo. The percentage of each cell cycle in the analyzed cell groups was calculated.

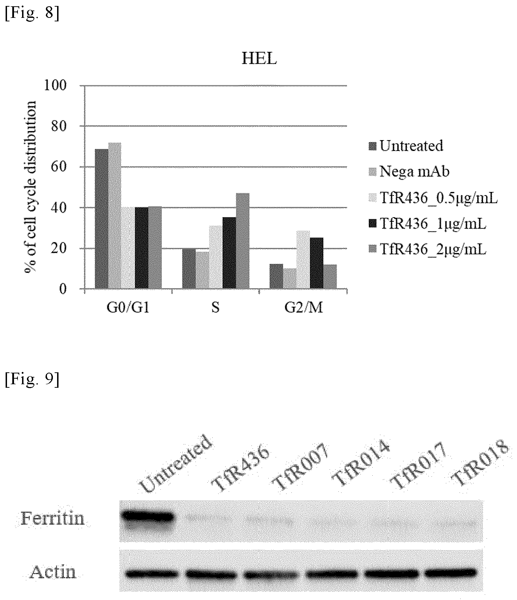

The results are shown in Fig. 8. It was confirmed that the TfR436 antibody increases the percentage of the cells of S-phase in the HEL cell line.

### Example 8: Cell death caused by TfR436 antibody

A change in the cell viability by exposure to the TfR436 antibody was verified. Specifically, MV4;11, Kasumi-1, and HNT-34 were each suspended in each type of optimal medium for cell culture to result in a cell density of 1.0 × 10⁵ cells/mL, and were then seeded in a T75 flask. The TfR436 antibody or the negative control monoclonal antibody (Nega mAb) was added to the flask to a final concentration of 5 µg/mL, and were then cultured at 37°C in a 5% CO₂ incubator. The cells were recovered at 48 and 96 hours after the addition of the antibody, and were then washed with PBS. Thereafter, the cells were re-suspended in 1 mL of PBS. The cell suspension and a trypan blue staining solution were mixed with each other at a ratio of 1 : 1, and the number of viable cells and the number of dead cells were counted using a blood cell counter. Besides, the cell suspension was diluted with PBS depending on the number of cells, and was adjusted so as not to interfere with the measurement with a blood cell counter. The percentage of viable cells to the total cell count was calculated as cell viability.

The results are shown in Table 1. It was confirmed that the TfR436 antibody decreases cell viability in MV4;11, Kasumi-1, and HNT-34.

### [Table 1]

**Table 1:**

| Cell Line | Antibody addition time (h) | Viability (%) | |
|---|---|---|---|
| | | Nega mAb | TfR436 |
| MV4;11 | 48 | 91.1 | 64.3 |
| | 96 | 93.8 | 34.8 |
| Kasumi-1 | 48 | 93.6 | 87.5 |
| | 96 | 92.5 | 65.4 |
| HNT-34 | 48 | 100.0 | 91.0 |
| | 96 | 97.1 | 84.4 |

### Example 9: Change in amount of ferritin caused by a plurality of TfR antibodies

A change in the amount of ferritin caused by exposure to a plurality of TfR antibodies (TfR007, TfR014, TfR017, and TfR018) was compared with that caused by the TfR436 antibody. Specifically, K562 was suspended in a medium for cell culture to result in a cell density of 1.0 × 10⁵ cells/mL, and was then seeded in a T75 flask. Each TfR antibody-added flask and an antibody-not-added flask were cultured at 37°C in a 5% CO₂ incubator. Besides, each TfR antibody was adjusted to a final concentration of 5 µg/mL. The cells were recovered at 72 hours after the addition of the antibody, and were then washed with PBS. Thereafter, each cell line (1.0 × 10⁶ cells) was suspended in 50 µL of Complete Lysis-M EDTA-free, and was then left at rest on ice for 30 minutes. After the cells had been centrifuged, a supernatant was recovered, and protein quantification was then carried out using Pierce BCA Protein Assay Kit. One-fifth volume of 5 × Sample Buffer (containing 2-mercaptoethanol) was added to and blended with the recovered supernatant, and the obtained mixture was then treated at 100°C for 3 minutes to prepare a sample. The prepared sample was applied in an amount of 12 µL (10 µg) each to a 5-20% gradient polyacrylamide gel, and was then electrophoresed. Thereafter, a protein was transcribed onto a PVDF membrane (Millipore #IPFL85R) by applying a voltage of 80 V for 60 minutes, using a transcription buffer (Tris-glycine buffer) containing 20% methanol. The transcribed PVDF membrane was blocked using a TBS blocking buffer (LI-COR #927-60001). Thereafter, the following primary antibodies were diluted with a blocking buffer, and were then incubated at 4°C overnight.

### Primary antibodies (clone names) (source, product number, dilution factor)

### (1) Ferritin Heavy chain (B-12) (Santa Cruz Biotechnology, #sc-376594, 1000-fold)

### (2) Anti-Actin, clone C4 (Merck, #MAB1501, 5000-fold)

After the incubation, the membrane was washed with 0.05% -PBST at room temperature for 5 minutes four times. The secondary antibody IRDye 800CW Goat anti-Mouse IgG (LI-COR, #925-32210) was 20,000-fold diluted with a blocking buffer, , followed by performing incubation at room temperature for 60 minutes.

After the incubation, the membrane was washed with 0.05% -PBST at room temperature for 5 minutes four times. Detection was carried out using Odyssey Fc Imaging System (LI-COR).

The results are shown in Fig. 9. It was confirmed that the plurality of TfR antibodies (TfR007, TfR014, TfR017, and TfR018) decrease the amount of ferritin in K562, as with the TfR436 antibody.

### Example 10: Change in amount of ROS in mitochondria caused by a plurality of TfR antibodies

A change in the amount of ROS in mitochondria caused by exposure to a plurality of TfR antibodies (TfR007, TfR014, TfR017, and TfR018) was compared with that caused by the TfR436 antibody. Specifically, MV4;11 was seeded at a cell density of 1 × 10⁵ cells/mL on a T25 flask. Each TfR antibody-added flask and an antibody-not-added flask were cultured at 37°C in a 5% CO₂ incubator. Besides, each TfR antibody was adjusted to a final concentration of 5 µg/mL. The cells were recovered at 72 hours after the addition of the antibody, and were then washed with HBSS twice, followed by cell counting. The cells were seeded at a cell density of 4 × 10⁵ cells/well on a V-bottom 96-well plate, and were then stained with 5 µM MitoSOX at 37°C for 10 minutes. The resulting cells were washed with HBSS twice, and were then transferred onto a plate for fluorescence measurement. The fluorescence was measured at Ex 515 nm and Em 600 nm, using SpectraMAX iD3 manufactured by MOLECULAR DEVICES. The fluorescence value of the solvent was subtracted from the measured value, and while the case of non-addition of the antibody was set to be 100%, the ROS production percentage in the case of addition of the TfR436 antibody was calculated.

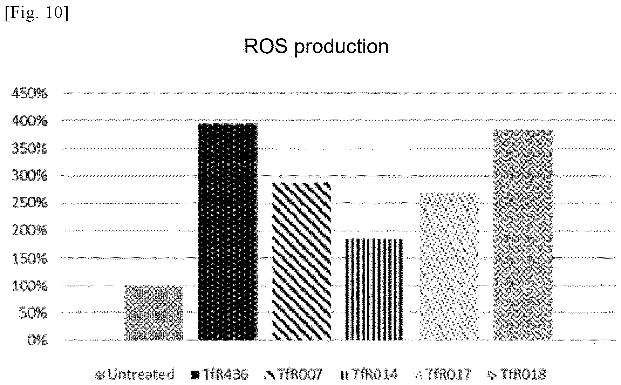

The results are shown in Fig. 10. It was confirmed that the plurality of TfR antibodies (TfR007, TfR014, TfR017, and TfR018) increase mitochondrial ROS production, as with the TfR436 antibody.

## Claims

1. An ROS production-enhancing agent, comprising a substance that recognizes a transferrin receptor.

2. A ferritin degradation-promoting agent, comprising a substance that recognizes a transferrin receptor.

3. A ferroptosis-inducing agent, comprising a substance that recognizes a transferrin receptor.

4. An apoptosis-inducing agent, comprising a substance that recognizes a transferrin receptor.

5. The agent according to any one of claims 1 to 4, wherein the substance that recognizes a transferrin receptor is an antibody that recognizes a transferrin receptor.

6. The agent according to claim 5, wherein the antibody that recognizes a transferrin receptor is an antibody that recognizes a human transferrin receptor.

7. The agent according to claim 6, wherein the antibody that recognizes a human transferrin receptor is an antibody that recognizes the amino acids at positions 629 to 633 of a human transferrin receptor, or an antibody that inhibits the binding of another antibody to the amino acids at positions 629 to 633 of a human transferrin receptor.

8. The agent according to any one of claims 5 to 7, wherein the antibody is an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and also having a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.

9. The agent according to any one of claims 5 to 8, wherein the antibody is an antibody having a heavy chain variable region as set forth in SEQ ID NO: 7 and a light chain variable region as set forth in SEQ ID NO: 8.

10. The agent according to any one of claims 5 to 7, wherein the antibody is any one of:
(a) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 11, 12, and 13, respectively, and also having a light chain first complementarity determining region (VL CDR1), a light chain second complementarity determining region (VL CDR2), and a light chain third complementarity determining region (VL CDR3), which are as set forth in SEQ ID NOs: 14, 15, and 16, respectively;
(b) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 17, 18, and 19, respectively, and also having a light chain first complementarity determining region (VL CDR1), a light chain second complementarity determining region (VL CDR2), and a light chain third complementarity determining region (VL CDR3), which are as set forth in SEQ ID NOs: 20, 21, and 22, respectively;
(c) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 23, 24, and 25, respectively, and also having a light chain first complementarity determining region (VL CDR1), a light chain second complementarity determining region (VL CDR2), and a light chain third complementarity determining region (VL CDR3), which are as set forth in SEQ ID NOs: 26, 27, and 28, respectively; and
(d) an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 29, 30, and 31, respectively, and also having a light chain first complementarity determining region (VL CDR1), a light chain second complementarity determining region (VL CDR2), and a light chain third complementarity determining region (VL CDR3), which are as set forth in SEQ ID NOs: 32, 33, and 34, respectively.

11. The agent according to claim 10, wherein the antibody is any one of:
an antibody having a heavy chain variable region as set forth in SEQ ID NO: 35 and a light chain variable region as set forth in SEQ ID NO: 36;
an antibody having a heavy chain variable region as set forth in SEQ ID NO: 37 and a light chain variable region as set forth in SEQ ID NO: 38;
an antibody having a heavy chain variable region as set forth in SEQ ID NO: 39 and a light chain variable region as set forth in SEQ ID NO: 40; and
an antibody having a heavy chain variable region as set forth in SEQ ID NO: 41 and a light chain variable region as set forth in SEQ ID NO: 42.

12. The agent according to any one of claims 5 to 11, wherein the antibody is a human antibody or a humanized antibody.

13. The agent according to any one of claims 5 to 12, wherein the antibody is an antibody fragment selected from the group consisting of Fab, Fab', F(ab')₂, a single-chain antibody (scFv), a multi-specific antibody, a disulfide-stabilized V region (dsFv) and a peptide comprising CDR.
